# EUROPÄISCHE PATENTSCHRIFT

(11) **EP 1 083 146 B1**
(45) Veröffentlichungstag und Bekanntmachung des Hinweises auf die Patenterteilung: **09.04.2003**
(21) Anmeldenummer: 00117681.7
(22) Anmeldetag: 17.08.2000
(51) Int. Cl.: C01B 33/18, B01J 12/02, B01J 19/24, B01J 19/26, B01J 37/00, C09C 3/00, A61K 7/00, C08K 3/22, D21H 17/69, C03B 19/10, A01N 1/00

(54) **Bakterizides, mit Silber dotiertes Siliciumdioxid**
Bactericide silver doped silica
Silice bactéricide dopée à l'argent

(30) Priorität: 09.09.1999 DE 19943057
(43) Veröffentlichungstag der Anmeldung: 14.03.2001
(73) Patentinhaber: Degussa AG, 40474 Düsseldorf (DE)
(72) Erfinder: Mangold, Helmut, Dr., 63517 Rodenbach (DE); Golchert, Rainer, 64807 Dieburg (DE)

(56) Entgegenhaltungen:
- EP-A- 0 850 876
- EP-A- 0 995 718
- US-A- 4 292 290
- DATABASE CHEMABS [Online] CHEMICAL ABSTRACTS SERVICE, COLUMBUS, OHIO, US; HONGO, AKIFUMI ET AL: "Optical waveguides and manufacture thereof" retrieved from STN Database accession no. 129:60395 CA XP002150667 & JP 10 133043 A (HITACHI CABLE, LTD., JAPAN) 22. Mai 1998 (1998-05-22)
- FORMENTI M ET AL: "PREPARATION IN A HYDROGEN-OXYGEN FLAME OF ULTRAFINE METAL OXIDE PARTICLES. OXIDATIVE PROPERTIES TOWARD HYDROCARBONS IN THE PRESENCE OF ULTRAVIOLET RADIATION" JOURNAL OF COLLOID AND INTERFACE SCIENCE,US,ACADEMIC PRESS, NEW YORK, NY, Bd. 39, Nr. 1, 1. April 1972 (1972-04-01), Seiten 79-89, XP002053084 ISSN: 0021-9797
- DATABASE WPI Section Ch, Week 199923 Derwent Publications Ltd., London, GB; Class D22, AN 1999-272888 XP002151264 & JP 11 086757 A (NIPPON ELECTRIC GLASS CO), 30. März 1999 (1999-03-30)
- PATENT ABSTRACTS OF JAPAN Bd. 1999, Nr. 08 30 Juni 1999 & JP 11 086 757 A (NIPPON ELECTRIC GLASS CO LTD) 30 M{rz 1999

## Beschreibung

Die Erfindung betrifft ein mittels Aerosol mit Silber oder Silberoxid dotiertes pyrogen hergestelltes Siliciumdioxid, das bakterizide Eigenschaften aufweist, sowie ein Verfahren zu seiner Herstellung sowie seiner Verwendung. Weiterhin betrifft sie seine Verwendung in Dispersionen oder als Füllstoff in Kautschuk und Silikonkautschuk.

Es ist bekannt pyrogen hergestellte Kieselsäure mit einem speziellen Verfahren in einem Schritt in der Flamme zu dotieren. (DE 196 50 500 A1). Dabei handelt es sich um eine Kombination einer Hochtemperaturflammenhydrolyse mit einer Pyrolyse. Von diesem Dotierverfahren zu unterscheiden ist das ältere sogenannte "co-fumed-Verfahren", bei der die gasförmigen Ausgangsprodukte (beispielsweise SiCl₄-Gas und AlCl₃-Gas) vorgemischt werden und gemeinsam in einem Flammenreaktor verbrannt werden.

Beim Dotierverfahren wird in eine Flamme, in der ein pyrogenes Oxid durch Flammenhydrolyse erzeugt wird, ein Aerosol eingespeist, wobei sich in diesem Aerosol ein Salz der zu dotierenden Verbindung befindet.

Es wurde nun gefunden, daß bei Verwendung von in Wasser gelösten Silbersalzen als Ausgangsprodukt für das Aerosol eine dotierte pyrogene Kieselsäure als Produkt anfällt, die bakterizide Eigenschaften aufweist.

Gegenstand der Erfindung ist eine mittels Aerosol mit Silber oder Silberoxid dotierte pyrogen hergestellte Kieselsäure, welche dadurch gekennzeichnet ist, daß die Basiskomponente eine pyrogen nach Art der Flammenoxidation oder bevorzugt der Flammenhydrolyse hergestellte Kieselsäure ist, die mit einer Dotierungskomponente von 1*10⁻⁴ und bis zu 20 Gew.% dotiert ist, wobei die Dotierungsmenge vorzugsweise im Bereich von 1 bis 10.000 ppm⁻ liegt und die Dotierungskomponente ein Salz oder eine Salzmischung einer Silberverbindung oder metallischen Silbers oder Mischungen davon ist, wobei die BET-Oberfläche des dotierten Oxids zwischen 1 und 600 m²/g liegt, bevorzugt im Bereich zwischen 40 und 400 m²/g.

Ein weiterer Gegenstand der Erfindung ist ein Verfahren zur Herstellung der mittels Aerosol mit Silber oder Silberoxid dotierten pyrogen hergestellten Kieselsäure, welches dadurch gekennzeichnet ist, daß man in eine Flamme, wie sie zur pyrogenen Herstellung von Kieselsäure nach Art der Flammenoxidation oder bevorzugt der Flammenhydrolyse benutzt wird, ein Aerosol einspeist, das Aerosol vor der Reaktion mit dem Gasgemisch der Flammenoxidation beziehungsweise der Flammenhydrolyse homogen mischt, dann das Aerosol-Gasgemisch in der Flamme abreagieren läßt und die entstandenen mit Silber oder Silberoxid dotierten pyrogen hergestellten Kieselsäuren in bekannter Weise vom Gasstrom abtrennt, wobei zur Herstellung des Aerosols eine wäßrige Lösung dient, die Salze oder Salzmischungen des Silbers oder das Metall selbst in gelöster oder suspendierter Form oder Mischungen davon enthält, wobei das Aerosol durch Vernebelung mittels einer Zweistoffdüse oder durch eine andere Art der Aerosolherstellung hergestellt wird.

Als Salze können eingesetzt werden: Ag(NO₃), Ag₂(SO₄), Ag₂O, auch komplexiert mit Komplexbildnern oder Ammoniak.

Das Verfahren der Flammenhydrolyse zur Herstellung von pyrogenen Oxiden ist aus Ullmanns Enzyklopädie der techn. Chemie 4. Aufl. Bd.21, S. 464 bekannt.

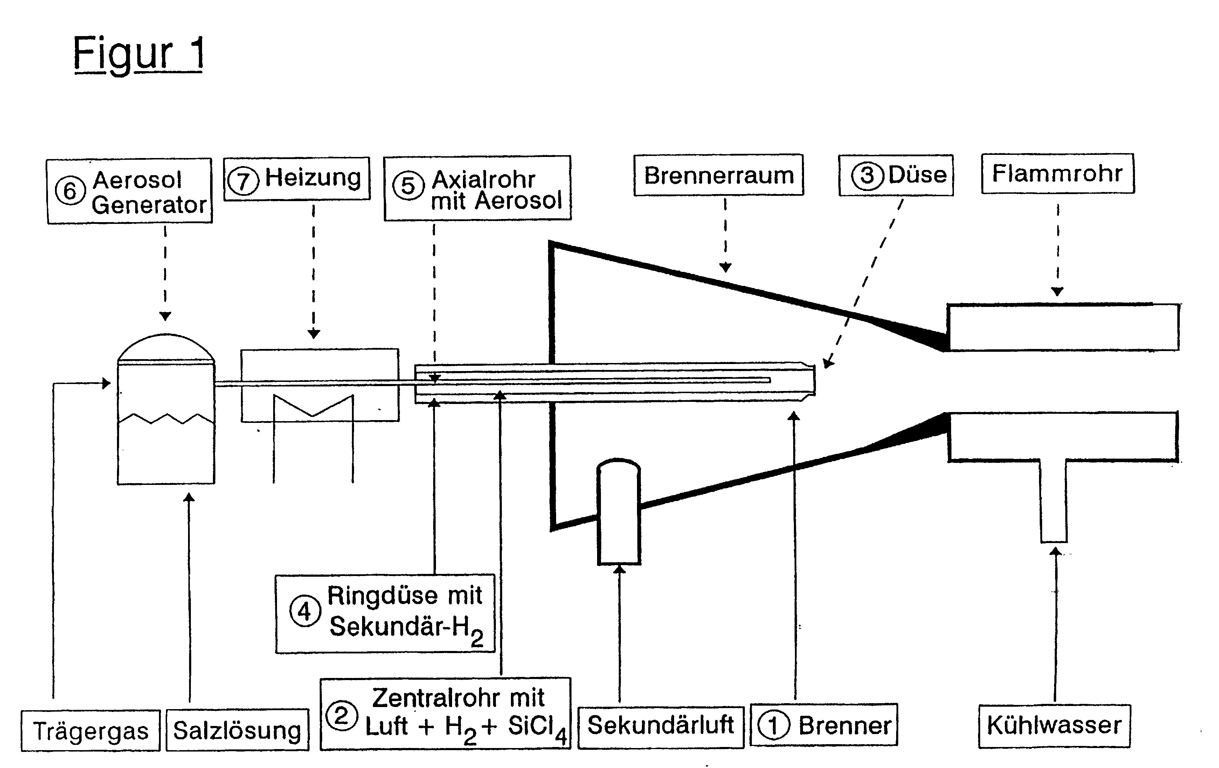

Das erfindungsgemäße dotierte Siliciumdioxid und das Verfahren zu seiner Herstellung sowie Verwendung werden anhand der Figur 1 und des folgenden Beispiels näher erläutert und beschrieben.

Figur 1 zeigt eine schematische. Darstellung der Dotierungsapparatur. Kernstück der Apparatur ist ein Brenner bekannter Bauart zur Herstellung von pyrogenen Oxiden. In Figur 1 ist der Brenner mit 1 bezeichnet.

Der Brenner 1 besteht aus einem Zentralrohr 2 , das in eine Düse 3 mündet, aus welcher der Hauptgasstrom in den Brennerraum strömt und dort abbrennt. Diese innere Düse ist von einer weiteren Ringdüse 4 umgeben, aus der (Ring- oder Sekundär-)Wasserstoff ausströmt.

Im Zentralrohr 2 befindet sich ein weiteres Axialrohr 5, das einige Zentimeter vor der Düse des Zentralrohrs 2 endet. In dieses Axialrohr 5 wird das Aerosol eingespeist.

Das Aerosol, das aus einer wäßrigen Silbersalzlösung besteht, wird in einem Aerosol-Generator 6 (Ultraschallvernebler) erzeugt.

Das in dem Aerosol-Generator erzeugte Silbersaiz-Wasser-Aerosol wird mittels eines leichten Traggasstromes durch eine Heizzone 7 geleitet, in der das mitgeführte Wasser verdampft, wobei in der Gasphase kleine Salzkristalle in feinverteilter Form zurückbleiben.

### Beispiel 1: Herstellung einer mit Silber oder Silberoxid dotierten pyrogenen Kieselsäure

4,44 kg/h SiCl₄ werden bei ca. 130 °C verdampft und in das Zentralrohr eines Brenners bekannter Bauart überführt. In das Zentralrohr werden zusätzlich 3 Nm³/h (Primär-) Wasserstoff und 7,5 Nm³/h Luft eingespeist.

Das Gasgemisch strömt aus der inneren Düse des Brenners und brennt in den Brennerraum und das daran anschließende wassergekühlte Flammrohr.

In die Manteldüse werden 0,5 Nm³/h (Mantel oder Sekundär-) Wasserstoff eingespeist.

In den Brennerraum werden noch zusätzlich 12 Nm³/h (Sekundär-)Luft eingespeist.

Aus dem Axialrohr 5 strömt die zweite Gaskomponente in das Zentralrohr 2.

Der zweite Gasstrom besteht aus einem Aerosol, das durch Ultraschallvernebelung in einer separaten Vernebelungseinheit erzeugt wird. Der Aerosolgenerator vernebelt dabei 725 g/h 5-prozentige wäßrige Silbersulfatlösung. Das Silbersulfataerosol wird mit Hilfe des Traggases von 0,5 Nm³/h Luft durch eine geheizte Leitung geführt, wobei das wäßrige Aerosol bei Temperaturen um ca. 180 °C in ein Gas und ein Salzkristall-Aerosol übergeht.

Die Reaktionsgase und die entstandene mit Silber oder Silberoxid dotierte pyrogene Kieselsäure werden durch Anlegen eines Unterdruckes durch ein Kühlsystem gesaugt und dabei der Partikel-Gasstrom auf ca. 100 bis 160 °C abgekühlt. In einem Filter oder Zyklon wird der Feststoff von dem Abgasstrom abgetrennt.

Die mit Silber(-oxid) dotierte pyrogen hergestellte Kieselsäure fällt als weißes feinteiliges Pulver an. In einem weiteren Schritt werden bei erhöhter Temperatur durch Behandlung mit wasserdampfhaltiger Luft noch anhaftende Salzsäurereste von der Kieselsäure entfernt.

Die BET-Oberfläche der mit Silber dotierten pyrogenen Kieselsäure beträgt 206 m²/g.

Die Herstellbedingungen sind in Tabelle 1 zusammengefaßt, weitere analytische Daten der so erhaltenen Kieselsäure sind in Tabelle 2 angegeben.

**Tabelle 1**

| Experimentelle Bedingungen bei der Herstellung von mit Silber(-oxid) dotierter pyrogener Kieselsäure | | | | | | | | |
|---|---|---|---|---|---|---|---|---|
| SiCl₄ kg/h | Primärluft Nm³/h | Sek-Luft Nm³/h | H₂ Kern Nm³/h | H₂ Mantel Nm³/h | Salzlösung | Aerosol menge kg/h | Luft Aeros. Nm³/h | BET m²/g |
| 4,44 | 7,5 | 12 | 3 | 0,5 | 5% wäßrige Ag₂SO4 | 0,725 | 0,5 | 206 |

Erläuterung: Primärluft = Luftmenge im Zentralrohr; Sek-Luft= Sekundärluft; H₂-Kern = Wasserstoff im Zentralrohr; Aerosolmenge = Massenstrom der in Aerosolform überführten Salzlösung; Luft-Aerosol = Traggasmenge (Luft) des Aerosols;

**Tabelle 2**

| Analytische Daten | | | | |
|---|---|---|---|---|
| | BET m²/g | pH-Wert 4-%Sus | Stampfdichte g/l | Ag₂O-Gehalt Gew.% |
| Beispiel | 206 | 4,13 | 24 | 1,72 |

Erläuterung: pH 4% Sus. = pH-Wert der vierprozentigen wäßrigen Suspension

### Vorteile der erfindungsgemäßen Kieselsäure

Die erfindungsgemäße Kieselsäure weist bakterizide Eigenschaften auf.

## Patentansprüche

1. Mittels Aerosol mit Silber oder Silberoxid dotierte pyrogen hergestellte Kieselsäure, **dadurch gekennzeichnet,daß** die Basiskomponente eine pyrogen nach der Art der Flammenoxidation oder bevorzugt der Flammenhydrolyse hergestellte Kieselsäure ist, die mit einer Dotierungskomponente von 0,0001 Gew.-% (=1x10⁻⁴ Gew.-%) und bis 20 Gew.% dotiert wurde, wobei die Dotierungsmenge vorzugsweise im Bereich von 1 bis 10.000 ppm liegt und die Dotierungskomponente ein Salz oder eine Salzmischung des Silbers oder eine Suspension einer Silberverbindung oder metallischen Silbers oder Mischungen davon ist, wobei die BET-Oberfläche des dotierten Oxids zwischen 1 und 600 m²/g liegt, bevorzugt im Bereich zwischen 40 und 400 m²/g.

2. Verfahren zur Herstellung von mittels Aerosol mit Silber oder Silberoxid pyrogenen dotierten Oxiden, **dadurch gekennzeichnet, daß** man in eine Flamme, wie sie zur Herstellung von pyrogener Kieselsäure nach der Art der Flammenoxidation oder bevorzugt der Flammenhydrolyse benutzt wird, ein Aerosol einspeist, dieses Aerosol vor der Reaktion mit dem Gasgemisch der Flammenoxidation bzw. Flammenhydrolyse homogen mischt, dann das Aerosol-Gasgemisch in einer Flamme abreagieren läßt und die entstandenen mit Silber oder Silberoxid dotierten pyrogenen Kieselsäuren in bekannter Weise vom Gasstrom abtrennt, wobei als Ausgangsprodukt des Aerosols eine Salzlösung dient, die Salze oder Salzmischungen des Silbers oder das Metall selbst in gelöster oder suspendierter Form oder Mischungen davon enthält, wobei das Aerosol durch Vernebelung mittels einer Zweistoffdüse oder durch eine andere Art der Aerosolherstellung vorzugsweise durch einen Aerosolgenerator nach der Ultraschallvernebelung hergestellt wird.

3. Verwendung der mittels Aerosol mit Silber oder Silberoxid dotierten pyrogenen Kieselsäure als bakteriziden Füllstoff insbesondere bei der Herstellung von Lacken oder Schichten, in der Papierindustrie, als katalytisch aktive Substanz, als Ausgangsmaterial zur Herstellung von Dispersionen, als Poliermaterial (CMP-Anwendungen), als keramischen Grundstoff, in der Elektronikindustrie, als Füllstoff für Polymere, als Ausgangsstoff zur Herstellung von Glas oder Glasbeschichtungen oder Glasfasern, als Trennhilfsmittel auch bei hohen Temperaturen, in der Kosmetikindustrie, als Absorbermaterial, als Additiv in der Silikon- und Kautschukindustrie, zur Einstellung der Rheologie von flüssigen Systemen, zur Hitzeschutzstabilisierung, als Wärmedämmaterial, als Fließhilfsmittel, als Füllstoff in der Dentalindustrie, als Hilfsstoff in der pharmazeutischen Industrie, in der Lackindustrie.

## Claims

1. A pyrogenically produced silica doped with silver or silver oxide by means of aerosol, **characterised in that** the base component is a silica produced pyrogenically by flame oxidation or preferably flame hydrolysis, which silica has been doped with a doping component of 0.0001 wt.% (=1x10⁻⁴ wt.%) and up to 20 wt.%, wherein the doping quantity preferably lies in the range from 1 to 10,000 ppm and the doping component is a salt or a salt mixture of silver or a suspension of a silver compound or metallic silver or mixtures thereof, wherein the BET surface area of the doped oxide lies between 1 and 600 m²/g, preferably in the range between 40 and 400 m²/g.

2. A method of producing pyrogenic oxides doped with silver or silver oxide by means of aerosol, **characterised in that** an aerosol is fed into a flame, as is used to produce pyrogenic silica by flame oxidation or preferably flame hydrolysis, said aerosol is mixed homogeneously prior to the reaction with the flame oxidation or flame hydrolysis gas mixture, then the aerosol/gas mixture is allowed to undergo reaction in a flame and the pyrogenic silicas doped with silver or silver oxide which are produced are separated in a known way from the gas stream, wherein a salt solution serves as the starting product for the aerosol, which salt solution contains salts or salt mixtures of the silver or the metal itself in dissolved or suspended form or mixtures thereof, wherein the aerosol is produced by nebulisation by means of a two-fluid nozzle or by another method of aerosol production, preferably by ultrasound nebulisation using an aerosol generator.

3. Use of the pyrogenic silica doped with silver or silver oxide by means of aerosol as a bactericidal filler in particular in the production of paints or coatings, in the paper industry, as a catalytically active substance, as a starting material for the production of dispersions, as a polishing material (CMP applications), as a ceramic basic substance, in the electronics industry, as a filler for polymers, as a starting material for the production of glass or glass coatings or glass fibres, as a release auxiliary even at high temperatures, in the cosmetics industry, as an absorber material, as an additive in the silicone and rubber industry, to adjust the rheology of liquid systems, for thermal stabilisation, as a heat insulating material, as a flow auxiliary, as a filler in the dental industry, as an auxiliary substance in the pharmaceuticals industry, or in the coatings industry.

## Revendications

1. Silice produite par voie pyrogène, dopée à l'argent ou à l'oxyde d'argent au moyen d'un aérosol,
**caractérisée en ce que**
le composant de base est une silice produite de façon pyrogène à la manière d'une oxydation à la flamme ou de préférence d'une hydrolyse à la flamme, qui a été dopée avec un composant dopant de 0,0001 % en poids (= 1 x 10⁻⁴ %) et jusqu'à 20 % en poids, la quantité pour le dopage se situant de préférence dans l'intervalle allant de 1 à 10 000 ppm et le composant de dopage étant un sel ou un mélange de sel d'argent ou une suspension d'un composé d'argent ou d'argent métallique, ou des mélanges de ces corps, la surface BET de l'oxyde dopé se situant entre 1 et 600 m²/g, de préférence dans un intervalle compris entre 40 et 400 m²/g.

2. Procédé de production par voie pyrogène d'oxydes dopés au moyen d'un aérosol avec de l'argent ou de l'oxyde d'argent,
**caractérisé en ce qu'**
on introduit un aérosol dans une flamme telle qu'on en utilise pour la production de silice pyrogène à la façon de l'oxyde à la flamme ou de préférence de l'hydrolyse à la flamme, on mélange de façon homogène cet aérosol avant la réaction avec le mélange gazeux de l'oxydation à la flamme ou selon les cas de l'hydrolyse à la flamme, puis on laisse réagir le mélange aérosol-gaz dans une flamme et on sépare de façon connue du courant gazeux les silices pyrogènes apparues dopées à l'argent ou à l'oxyde d'argent, opération dans laquelle on utilise comme produit de départ de l'aérosol une solution de sel, les sels ou les mélanges de sels d'argent ou du métal lui-même ou des mélanges de ces corps, étant contenus sous forme dissoute ou en suspension, l'aérosol étant produit par nébulisation au moyen d'une buse binaire ou par un autre type de production d'aérosol, de préférence par un générateur d'aérosol selon une nébulisation à ultrasons.

3. Utilisation de silice pyrogène dopée à l'argent ou à l'oxyde d'argent au moyen d'un aérosol, comme charge bactéricide en particulier dans la fabrication de vernis ou d'enduits, dans l'industrie du papier, comme substance à action catalytique, comme produit de départ pour la production de dispersions, comme matériau de polissage (applications CMP), comme après céramique, dans l'industrie électronique, comme charge pour polymères, comme produit de départ pour la fabrication de verre, de revêtements de verre ou de fibres de verre, comme adjuvant de séparation même à haute température, dans l'industrie cosmétique, comme matériau absorbant, comme additif dans l'industrie des silicones et du caoutchouc, pour le réglage de la rhéologie des systèmes liquides, la stabilisation de la protection thermique, comme matériau isolant thermique, comme adjuvant de fluidité, comme charge dans l'industrie dentaire, comme adjuvant pharmaceutique, et dans l'industrie des vernis.
